# FASCICULE DE BREVET EUROPEEN

(11) **EP 1 110 538 B1**
(45) Date de publication et mention de la délivrance du brevet: **10.05.2006**
(21) Numéro de dépôt: 00403333.8
(22) Date de dépôt: 29.11.2000
(51) Int. Cl.: A61K 8/34, A61Q 19/02, A61Q 5/08

(54) **Composition cosmétique comprenant un dérivé d'aminophénol**
Aminophenolderivat enthaltendes Kosmetikum
Cosmetic composition comprising an aminophenol derivative

(30) Priorité: 20.12.1999 FR 9916075
(43) Date de publication de la demande: 27.06.2001
(73) Titulaire: L'OREAL, 75008 Paris (FR)
(72) Inventeur: Chevalier, Véronique, 94440 Villecresnes (FR); Quest, Mélanie, 75005 Paris (FR)
(74) Mandataire: Kromer, Christophe

(56) Documents cités:
- EP-A- 0 898 956
- WO-A-99/10318
- DATABASE WPI Week 9722 Derwent Publications Ltd., London, GB; AN 1997-241638 XP002086051 "Skin external agents used for whitening skin - contains 4-aminophenol and eg. polyoxyethylene" & JP 09 077655 A (MIKIMOTO SAIYAKU), 25 mars 1997 (1997-03-25)
- PATENT ABSTRACTS OF JAPAN vol. 1995, no. 06, 31 juillet 1995 (1995-07-31) & JP 07 061905 A (MIKIMOTO PHARMACEUT CO LTD), 7 mars 1995 (1995-03-07)
- SAKUMA K ET AL: "RELATIONSHIP BETWEEN TYROSINASE INHIBITORY ACTION AND OXIDATION- REDUCTION POTENTIAL OF COSMETIC WHITENING INGREDIENTS AND PHENOL DERIVATIVES" ARCHIVES OF PHARMACAL RESEARCH,KR,NATL. FISHERIES UNIVERSITY, PUSAN, vol. 22, no. 4, août 1999 (1999-08), pages 335-339, XP000882119 ISSN: 0253-6269

## Description

L'invention concerne une composition, notamment cosmétique, comprenant un dérivé d'aminophénol, ainsi que ses utilisations. Elle concerne également un procédé de solubilisation d'un dérivé d'aminophénol.

Depuis plusieurs années, des efforts importants ont été déployés en vue de proposer des substances dépigmentantes topiques inoffensives présentant une bonne efficacité.

Ces substances sont particulièrement recherchées en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Divers agents dépigmentants ont ainsi été proposés dans l'art antérieur. En particulier, il a été démontré par la Demanderesse que certains dérivés d'aminophénol avaient la propriété d'inhiber la mélanogénèse même à faibles concentrations, sans faire preuve de cytotoxicité. Ces composés, ainsi que leur mode de préparation, sont décrits dans la demande de brevet WO 99/10318.

Ces composés sont des aminophénols présentant une chaîne hydrocarbonée plus ou moins longue, de préférence alcoxycarbonyle, liée à l'atome d'azote. Ils présentent l'inconvénient d'être peu, voire pas du tout, solubles dans l'eau. Leur introduction dans des compositions cosmétiques nécessite, pour les composés à courte chaîne hydrocarbonée, une mise en solution hydroalcoolique, qui n'est pas toujours souhaitable lorsque la composition est destinée, par exemple, à être appliquée sur le contour de l'oeil. En outre, on a remarqué que les gels hydroalcooliques avaient tendance à piéger ces composés dans leur réseau, limitant ainsi leur diffusion dans l'épiderme malpighien.

De leur côté, les composés à longue chaîne hydrocarbonée sont insolubles dans les huiles, en raison de leur encombrement stérique, et ont tendance à recristalliser dans l'eau. Dans la demande de brevet FR-99 09663, il a été proposé de les incorporer dans la bicouche de vésicules lipidiques afin de faciliter leur formulation dans des produits cosmétiques. Toutefois, cette inclusion implique une mise en oeuvre parfois délicate.

Il reste donc nécessaire de pouvoir solubiliser aisément ces dérivés d'aminophénol dans un milieu physiologiquement acceptable qui entraîne un minimum d'inconfort à l'application sur la peau ou le cuir chevelu, et qui soit facile à mettre en oeuvre. En outre, il est nécessaire de pouvoir solubiliser une quantité suffisante de ces composés en vue d'une utilisation cosmétique ou dermatologique, sans recristallisation de ces composés ni perte de stabilité de la composition les contenant. Cette instabilité résulterait en effet en une perte d'efficacité plus ou moins importante de ces compositions et/ou en une modification de leur aspect, qui risquerait de détourner l'utilisateur de celles-ci.

Or, la Demanderesse a maintenant découvert que ces dérivés d'aminophénol pouvaient être solubilisés dans les alcools gras oxyalkylénés.

La présente invention a donc pour objet une composition comprenant, dans un milieu physiologiquement acceptable, un composé solubilisant ne se trouvant pas sous forme de vésicules, choisi parmi :
les alcools gras oxyalkylénés,

L'invention a également pour objet un procédé de solubilisation d'au moins un dérivé d'aminophénol de formule (I) ci-dessus comprenant l'étape consistant à le mélanger à au moins un solubilisant tel que défini ci-dessus.

Le N-cholestéryloxycarbonyl-4-para-aminophénol , ainsi que son procédé de préparation, sont décrits dans la demande de brevet WO 99/10318 dont l'enseignement est incorporé ici par référence.

Comme alcools gras utilisables selon l'invention, on préfère les alcools gras oxyéthylénés et/ou oxypropylénés, de préférence saturés, dont la chaîne hydrocarbonée renferme avantageusement de 8 à 30 atomes de carbone. Des exemples d'alcools gras oxyalkylénés sont constitués par l'alcool cétylique oxyéthyléné (20 OE) oxypropyléné (5 OP), l'alcool laurylique oxyéthyléné comprenant 4 ou 23 motifs d'oxyde d'éthylène et le mélange d'alcools cétylique et stéarylique oxyéthylénés, comprenant 20 motifs d'oxyde d'éthylène.

Ces solubilisants sont oxyalkylénés, c'est-à-dire généralement oxyéthylénés et/ou oxypropylénés. Il a en effet été découvert que ces solubilisants permettaient, de façon surprenante, de solubiliser une quantité de dérivé d'aminophénol au moins égale à 10% en poids, par rapport au poids total du solubilisant utilisé, à une température généralement inférieure à 80°C, sans entraîner de recristallisation du dérivé d'aminophénol à température ambiante.

Les solubilisants définis ci-dessus peuvent être utilisés pour solubiliser le N-cholestéryloxycarbonyl-4-para-aminophénol avant de l'introduire, indifféremment, dans la phase huileuse ou dans la phase aqueuse de la composition selon l'invention.

Cette composition peut renfermer de 0,01 à 15% en poids, préférentiellement de 0,5 à 5% en poids de N-cholestéryloxycarbonyl-4-para-aminophénol, par rapport au poids total de la composition. La quantité de solubilisant dépendra de la quantité de dérivé d'aminophénol à solubiliser et pourra par exemple aller de 0,05 à 25% en poids, préférentiellement de 2 à 20% en poids par rapport au poids total de la composition.

La composition selon l'invention est plus particulièrement destinée à une application topique sur la peau ou les cheveux et elle comprend donc un milieu physiologiquement acceptable, c'est-à-dire compatible avec la peau et/ou ses phanères (ongles, poils, cheveux).

Elle peut se présenter sous toutes les formes galéniques normalement utilisées pour une application topique, notamment sous forme d'une solution aqueuse, hydroalcoolique, d'une émulsion huile-dans-eau ou eau-dans-huile ou multiple, d'un gel aqueux ou huileux, ou d'un produit anhydre liquide, pâteux ou solide.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou les cheveux sous forme d'aérosol. Elle peut également se présenter sous forme solide, et par exemple sous forme de stick.

De façon connue, la composition selon l'invention peut contenir également les adjuvants habituels dans les domaines cosmétique et dermatologique, tels que des gélifiants hydrophiles ou lipophiles, des actifs hydrophiles ou lipophiles, des conservateurs, des antioxydants, des solvants, des parfums, des charges, des filtres, des pigments, des absorbeurs d'odeur et des matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans des vésicules lipidiques et/ou dans des nanoparticules.

Bien entendu, l'homme du métier veillera à choisir ces éventuels composés complémentaires, actifs ou non actifs, et/ou leur quantité, de telle manière que les propriétés avantageuses du N-cholestéryloxycarbonyl-4-para-aminophénol ne soient pas, ou sensiblement pas, altérées par l'adjonction envisagée.

Lorsque la composition selon l'invention est une émulsion, la proportion de la phase grasse peut aller de 0,5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple, outre le solubilisant du dérivé d'aminophénol, les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les agents kératolytiques et/ou desquamants, les filtres UV, les agents anti-irritants et apaisants et leurs mélanges. En outre, bien que le dérivé d'aminophénol ait à lui seul une activité dépigmentante justifiant son utilisation comme seul actif dépigmentant d'une composition blanchissante, on peut également ajouter à la composition selon l'invention d'autres agents dépigmentants, ce qui permet d'utiliser ces derniers à des doses moins élevées. En cas d'incompatibilité, certains au moins des actifs peuvent être incorporés dans des sphérules, notamment des vésicules ioniques ou non-ioniques et/ou des nanoparticules (nanocapsules et/ou nanosphères), de manière à ce que les actifs incompatibles entre eux ou avec le N-cholestéryloxycarbonyl-4-para-aminophénol soient isolés les uns des autres dans la composition.

La composition selon l'invention peut constituer notamment des produits de protection, de soin ou de maquillage pour le visage, pour le cou, pour les mains ou pour le corps. Elle peut en variante se présenter sous forme de produit capillaire, en particulier de shampooing ou d'après-shampooing.

Cette composition est particulièrement bien adaptée à une utilisation par voie topique, en vue de blanchir et/ou dépigmenter la peau, les poils et/ou les cheveux.

La présente invention concerne donc également un procédé cosmétique de dépigmentation et/ou blanchiment de la peau humaine, des poils et/ou des cheveux, caractérisé en ce qu'il consiste à appliquer sur la peau, les poils et/ou les cheveux une composition telle que décrite précédemment.

La présente invention concerne enfin l'utilisation de la composition définie précédemment pour la fabrication d'une préparation destinée à dépigmenter et/ou blanchir la peau, les poils et/ou les cheveux.

L'invention sera maintenant illustrée à l'aide des exemples non limitatifs suivants. Sauf indication contraire, les quantités sont données en pourcentage pondéral.

### Exemple 1: Emulsion fluide

### Phase A

- N-cholestéryloxycarbonyl-4-para-aminophénol 0,5 %
- Mélange d'alcools cétylique et stéarylique oxyéthylénés (20 OE) 2 %
- Huile de ricin hydrogénée oxyéthylénée (60 OE) 2,5 %

### Phase A'

- Huile d'abricot 5 %
- Filtres UV 5,6 %
- Cyclohexasiloxane 5 %
- Sesquistéarate de méthylglucose polyéthoxylé (20 OE) 2 %
- Sesquistéarate de méthylglucose 2 %

### Phase B

- Conservateurs 0,65 %
- Triéthanolamine 1,35 %
- EDTA disodique 0,05 %
- Glycérine 3 %
- Eau déminéralisée qsp. 100 %

### Phase C

- Gélifiants 2,25 %

Cette composition est préparée de la manière suivante : la phase A est d'abord chauffée à environ 80°C jusqu'à dissolution complète, puis introduite dans la phase A' préalablement portée à la même température. Le mélange est ensuite ajouté à la phase B chauffée à environ 80°C pour émulsification, puis la phase C est incorporée à environ 50°C à l'émulsion H/E ainsi obtenue.

Elle permet d'estomper les taches pigmentaires sur le visage, le décolleté et les mains.

Après deux mois de conservation à température ambiante et à 45°C, on n'a pas observé de modification de la viscosité ou de la couleur de la composition, ni de dégagement d'odeur. En outre, on n'a pas noté de diminution de la teneur en N-cholestéryloxycarbonyl-4-para-aminophénol, telle que déterminée par HPLC, ni observé de formation de cristaux de ce composé.

### Exemple 2: Comparaison des solubilisants

On a comparé, dans le Tableau 1 ci-après, la quantité de (N-cholestéryloxycarbonyl-4-para-aminophénol, ou CPAP) susceptible d'être solubilisée dans divers agents solubilisants, à 60°C.

**Tableau 1 Solubilisation de CPAP par les alcools**

| **Solubilisant** | **Quantité de CPAP solubilisée** | |
|---|---|---|
| Laureth-23 | 4,8% | |
| Laureth-4 | 4,8% | |
| Alcool cétylique oxyéthyléné (20 OE) oxypropyléné (5 OP) | 14,2% | |
| Octyldodécanol | 0,5% | |
| Hexyldécanol | insoluble | |

Il ressort du Tableau 1 ci-dessus que les alcools gras non oxyalkylénés ne permettent pas, ou peu, de solubiliser le N-cholestéryloxycarbonyl-4-para-aminophénol selon l'invention.

La Demanderesse a en outre observé que le mélange d'alcool butylique oxyéthyléné (26 OE) oxypropyléné (26 OP) et d'huile de ricin hydrogénée oxyéthylénée (40 OE) permettait de solubiliser 14,2% de CPAP.

## Revendications

1. Composition comprenant, dans un milieu physiologiquement acceptable, un composé solubilisant ne se trouvant pas sous forme de vésicules choisi parmi les alcools gras oxyalkylénés, et le N-cholestéryloxycarbonyl-4-para-aminophénol.

2. Composition selon la revendication précédente, **caractérisée en ce que** N-cholestéryloxycarbonyl-4-para-aminophénol représente de 0,01 à 15% en poids, préférentiellement de 0,5 à 5% en poids, par rapport au poids total de la composition.

3. Composition selon la revendication 1 ou 2, **caractérisée en ce que** ledit solubilisant est choisi parmi les alcools gras oxyéthylénés et/ou oxypropylénés.

4. Composition selon l'une quelconque des revendications 1 à 3, **caractérisée en ce que** ledit solubilisant représente de 0,05 à 25% en poids, et préférentiellement de 2 à 20% en poids, par rapport au poids total de la composition.

5. Procédé cosmétique de dépigmentation et/ou blanchiment de la peau humaine, des poils et/ou des cheveux, **caractérisé en ce qu'**il consiste à appliquer sur la peau, les poils et/ou les cheveux une composition selon l'une quelconque des revendications 1 à 4.

6. Utilisation de la composition selon l'une quelconque des revendications 1 à 4 pour la fabrication d'une préparation destinée à dépigmenter et/ou blanchir la peau, les poils et/ou les cheveux.

7. Procédé de solubilisation du N-cholestéryloxycarbonyl-4-para-aminophénol comprenant l'étape consistant à le mélanger à au moins un solubilisant choisi parmi les alcools gras oxyalkylénés.

## Patentansprüche

1. Zusammensetzung, die in einem physiologisch akzeptablen Medium eine solubilisierende Verbindung, die nicht in Form von Vesikeln vorliegt und unter den alkoxylierten Fettalkoholen ausgewählt ist, und N-Cholesteryloxycarbonyl-4-paraaminophenol enthält.

2. Zusammensetzung nach dem vorhergehenden Anspruch, **dadurch gekennzeichnet, dass** das N-Cholesteryloxycarbonyl-4-para-aminophenol 0,01 bis 15 Gew.-% und vorzugsweise 0,5 bis 5 Gew.-%, bezogen auf das Gesamtgewicht der Zusammensetzung, ausmacht.

3. Zusammensetzung nach Anspruch 1 oder 2, **dadurch gekennzeichnet, dass** das Solubilisierungsmittel unter den ethoxylierten und/oder propoxylierten Fettalkoholen ausgewählt ist.

4. Zusammensetzung nach einem der Ansprüche 1 bis 3, **dadurch gekennzeichnet, dass** das Solubilisierungsmittel 0,05 bis 25 Gew.-% und vorzugsweise 2 bis 20 Gew.-%, bezogen auf das Gesamtgewicht der Zusammensetzung, ausmacht.

5. Kosmetisches Verfahren zur Depigmentierung und/oder zum Bleichen der menschlichen Haut, der Körperhaare und/oder der Haare, **dadurch gekennzeichnet, dass** es darin besteht, auf die Haut, die Körperhaare und/oder die Haare eine Zusammensetzung nach einem der Ansprüche 1 bis 4 aufzutragen.

6. Verwendung der Zusammensetzung nach einem der Ansprüche 1 bis 4 für die Herstellung eines Präparats, das dazu vorgesehen ist, die Haut, die Körperhaare und/oder die Haare zu bleichen und/oder zu depigmentieren.

7. Verfahren zur Solubilisierung von N-Cholesteryloxycarbonyl-4-para-aminophenol, das einen Schritt umfasst, der darin besteht, es mit mindestens einem Solubilisierungsmittel zu vermischen, das unter den alkoxylierten Fettalkoholen ausgewählt ist.

## Claims

1. Composition comprising, in a physiologically acceptable medium, a solubilizing compound which is not in the form of vesicles, chosen from oxyalkylenated fatty alcohols, and N-cholesteryloxycarbonyl-4-para-aminophenol.

2. Composition according to the preceding claim, **characterized in that** N-cholesteryloxycarbonyl-4-para-aminophenol represents from 0.01% to 15% by weight and preferably from 0.5% to 5% by weight relative to the total weight of the composition.

3. Composition according to Claim 1 or 2, **characterized in that** the said solubilizing agent is chosen from oxyethylenated and/or oxypropylenated fatty alcohols.

4. Composition according to any one of Claims 1 to 3, **characterized in that** the said solubilizing agent represents from 0.05% to 25% by weight and preferably from 2% to 20% by weight relative to the total weight of the composition.

5. Cosmetic process for depigmenting and/or bleaching human skin, body hairs and/or head hair, **characterized in that** it consists in applying a composition according to any one of Claims 1 to 4 to the skin, body hairs and/or head hair.

6. Use of the composition according to any one of Claims 1 to 4 to manufacture a preparation intended for depigmenting and/or bleaching the skin, body hairs and/or head hair.

7. Process for dissolving N-cholesteryloxycarbonyl-4-para-aminophenol, comprising the step consisting in mixing it with at least one solubilizing agent chosen from oxyalkylenated fatty alcohols.
